# FASCICULE DE BREVET EUROPEEN

(11) **EP 1 090 653 B1**
(45) Date de publication et mention de la délivrance du brevet: **05.12.2001**
(21) Numéro de dépôt: 00390015.6
(22) Date de dépôt: 15.09.2000
(51) Int. Cl.: A61M 5/32

(54) **Dispositif d'injection à usage unique**
Injektionsvorrichtung für Einmalverwendung
Single-use injection device

(30) Priorité: 07.10.1999 FR 9912500
(43) Date de publication de la demande: 11.04.2001
(73) Titulaire: SANOFI-SYNTHELABO, 75013 Paris (FR)
(72) Inventeur: Brunel, Marc, 31000 Toulouse (FR)
(74) Mandataire: Cabinet BARRE LAFORGUE & associés

(56) Documents cités:
- WO-A-92/18187
- WO-A-98/48869
- WO-A-99/47194

## Description

L'invention concerne un dispositif d'injection à usage unique conçu pour être pré-rempli d'une dose de liquide à injecter.

Les dispositifs d'injection à usage unique utilisés traditionnellement à l'heure actuelle consistent en des seringues pré-remplies dotées d'un capuchon destiné à protéger l'aiguille avant utilisation, puis retiré en vue de l'injection, et enfin remis en place après injection en vue d'éviter les risques ultérieurs de piqûre avec l'aiguille souillée.

Il s'est toutefois avéré qu'une telle conception présente un inconvénient majeur résultant du fait que, lors du recapuchonnage de l'aiguille, le capuchon doit être présenté en regard de l'extrémité de cette aiguille. Cette obligation s'est en effet avérée être la cause d'accidents par piqûre relativement fréquents avec tous les risques que comportent de telles piqûres avec une aiguille souillée. De plus, de telles seringues ne présentent aucune sécurité visant à interdire leur réutilisation et doivent donc être l'objet de soins particuliers en vue de leur destruction.

En vue de pallier ces inconvénients, la technique habituelle consiste à doter les dispositifs d'injection d'un étui protecteur apte à coulisser le long du corps de seringue ou à l'intérieur duquel la seringue peut se rétracter, de façon à obtenir soit une position d'injection où l'aiguille d'injection est dégagée, soit une position de protection après usage où cette aiguille d'injection est logée et protégée à l'intérieur de l'étui protecteur.

Selon ce principe, une solution classique consiste à réaliser des dispositifs d'injection comportant des moyens élastiques interposés entre le corps de seringue et l'étui protecteur, agencés pour être maintenus comprimés et assurer un positionnement de l'étui protecteur et de la seringue dans leur position d'injection, avant et en cours d'injection, et pour se détendre et engendrer automatiquement le coulissement dudit étui protecteur ou de ladite seringue vers leur position de protection après usage, en fin d'injection.

Cette solution permet de réaliser des dispositifs d'injection tels que décrits dans la demande de brevet internationale WO-93.25254 et européenne EP-A-0307367, d'une part garantissant contre tout risque de piqûre accidentelle avant et après injection, du fait notamment que l'étui protecteur ou la seringue sont amenés à coulisser de façon automatique grâce au déclenchement des moyens élastiques, après injection, et d'autre part empêchant toute réutilisation de la seringue.

Le document WO-A-98 48869 décrit une seringue qui comprend un corps portant une aiguille, délimitant une chambre pour une dose de liquide et étant obturé par un piston solidaire d'une tige de piston. L'aiguille est précontrainte par un ressort mais tenue par moyens de blocage. Après déclenchement de l'aiguille par le bout de la tige de piston (c'est à dire après l'injection), le piston et l'aiguille sont repoussés par le ressort dans un creux de la tige de piston.

Toutefois, il s'avère que ces dispositifs d'injection nécessitent la réalisation de seringues spécifiques donc d'un coût notablement supérieur à celui des seringues classiques telles que les seringues en verre produites actuellement en très grande série à un coût très faible.

En effet, aucune solution n'a été trouvée au problème qui consiste à obtenir un dispositif d'injection de ce type non réutilisable après injection, équipé d'une seringue classique.

Les dispositifs d'injection actuels de ce type présentent également un autre inconvénient provenant du fait que lors du déclenchement des moyens élastiques, commandé par des moyens de déverrouillage disposés sur la tige de piston, il n'est aucunement garanti que la seringue a été entièrement vidée du fait des tolérances admises concernant la longueur des seringues lors de la fabrication de ces dernières. Or, de tels dispositifs d'injection étant notamment destinés à être utilisés en vue de l'injection de très faibles doses de liquides (de l'ordre du millimètre cube) dont la quantité administrée doit être rigoureusement respectée, cette solution technique s'avère inapte à satisfaire les exigences requises.

La présente invention vise à pallier ces inconvénients et a pour objectif essentiel de fournir un dispositif d'injection de fabrication aisée comportant une seringue classique, et non réutilisable après injection.

Un autre objectif de l'invention est de fournir un dispositif d'injection garantissant l'injection totale de la dose de liquide.

A cet effet, l'invention vise un dispositif d'injection à usage unique comprenant une seringue dotée d'un nez antérieur portant une aiguille d'injection et d'une collerette postérieure, ladite seringue délimitant une chambre remplie d'une dose de liquide à injecter, obturée par un piston solidarisé à une des extrémités d'une tige de piston dotée d'un poussoir au niveau de son extrémité opposée.

Selon l'invention, ce dispositif d'injection comprend un dispositif de protection après usage de la seringue comportant :
- un étui protecteur composé de deux corps tubulaires, dits corps antérieur et corps postérieur, dotés de moyens d'assemblage aptes à permettre de les solidariser dans le prolongement l'un de l'autre, le corps antérieur présentant un diamètre conjugué de celui de la seringue et une longueur adaptée pour loger partiellement ladite seringue, et le corps postérieur présentant un diamètre supérieur à celui de la collerette de ladite seringue, et comportant des organes d'appui-doigts,
- une bague disposée dans le corps postérieur et comportant des moyens de blocage de la collerette de la seringue aptes à permettre d'introduire ladite seringue en présentant l'aiguille en regard du corps postérieur de l'étui protecteur,
- la bague et le corps postérieur comportant :
   . des premiers moyens de blocage relatif en translation aptes à permettre d'introduire ladite bague dans ledit corps postérieur en la présentant en regard de la face d'assemblage de ce dernier avec le corps antérieur, et définissant une première position de blocage, dite d'injection, permettant l'injection une fois le dispositif d'injection assemblé,
   . des deuxièmes moyens de blocage relatif en translation définissant une deuxième position de blocage, dite de protection après usage, obtenue après coulissement vers l'arrière de la bague à l'intérieur du corps postérieur, dans laquelle l'aiguille d'injection se trouve logée dans le corps antérieur,
- la bague et le corps antérieur étant dotés de moyens de butée aptes à maintenir des moyens élastiques comprimés dans la première position de blocage d'injection,
- et la bague ou le corps postérieur comportant une portion postérieure déformable radialement et portant les premiers moyens de blocage en translation, agencée pour être déformée par le poussoir de la tige de piston en fin de course de cette dernière, de façon à libérer lesdits premiers organes de blocage et à autoriser le déplacement de la bague vers sa deuxième position de blocage de protection après usage.

Le dispositif de protection de seringues selon l'invention comporte donc quatre éléments principaux (bague, corps antérieur et postérieur de l'étui protecteur, et moyens élastiques) conçus pour être assemblés de façon à constituer un ensemble pré-monté dans lequel on vient ensuite insérer une seringue classique, et qui assure ensuite la protection de l'aiguille après usage et empêche toute réutilisation.

De plus, l'assemblage du dispositif de protection est réalisé de façon très simple par étapes facilement automatisables. Cet assemblage est, en effet, réalisé selon les étapes suivantes :
- introduction de la bague dans le corps postérieur de l'étui protecteur jusqu'à la positionner dans la première position de blocage,
- mise en place des moyens élastiques non comprimés dans la bague,
- assemblage des corps postérieur et antérieur conduisant à la compression des moyens élastiques par les moyens de butée dudit corps antérieur et de la bague.

Cet ensemble pré-monté permet de mettre en place ultérieurement une seringue pré-remplie classique qui se trouve bloquée lors de cette mise en place au niveau de sa collerette, dans sa position d'injection, puis est ramenée automatiquement, après injection, dans une position de protection interdisant toute réutilisation.

Selon un mode de mise en oeuvre préférentiel, la bague est réalisée en un matériau de couleur différente de celui de l'étui protecteur, de façon, à des fins notamment de sécurité, à permettre d'identifier un dispositif d'injection déjà utilisé.

De plus, de façon avantageuse, le corps antérieur de l'étui protecteur est réalisé en un matériau translucide, de façon à permettre de visualiser la seringue.

Le corps postérieur de l'étui protecteur est, quant à lui, avantageusement réalisé en un matériau opaque de façon à masquer à la vue le mécanisme de déclenchement susceptible de troubler le praticien et le patient.

Par ailleurs, et de façon avantageuse, les moyens de blocage en translation de la bague et du corps postérieur de l'étui protecteur comprennent :
- au moins une rainure longitudinale interne ménagée dans le corps postérieur, prolongée par une cavité de blocage postérieure,
- pour chaque rainure, deux crans de blocage postérieur et antérieur ménagés sur la bague et adaptés pour coulisser dans ladite rainure et se loger dans la cavité de blocage.

Cette disposition conduit, outre à définir les première et deuxième position de blocage en translation, à bloquer relativement en rotation la bague et le corps postérieur.

De plus, chaque cavité de blocage consiste avantageusement en une lumière ménagée dans la paroi périphérique du corps postérieur, délimitée postérieurement par un épaulement interne et antérieurement par un cran interne en forme de dent asymétrique dotée d'une face antérieure inclinée, et portée par une languette déformable ménagée dans ladite paroi périphérique du corps postérieur.

Par ailleurs, selon un mode de réalisation préférentiel, les moyens d'assemblage des corps antérieur et postérieur de l'étui protecteur comprennent une gorge annulaire interne ménagée dans le corps postérieur et un jonc de clipsage externe ménagé sur le corps antérieur.

De plus, de façon avantageuse, le corps antérieur de l'étui protecteur comporte, pour chaque rainure du corps postérieur, un ergot externe apte à se loger dans ladite rainure. Cette disposition conduit à l'obtention d'un blocage relatif en rotation des deux corps de l'étui protecteur.

Les moyens de blocage de la collerette de la seringue comprennent quant à eux, avantageusement, au moins un cran interne postérieur ménagé dans la bague et consistant en une dent asymétrique dotée d'une face postérieure inclinée, et un épaulement interne antérieur ménagé dans la bague à une distance de chaque cran équivalent à l'épaisseur de ladite collerette.

Quant aux moyens de butée des moyens élastiques, ils comportent avantageusement l'épaulement précité ménagé dans la bague et un épaulement interne ménagé dans le corps antérieur au niveau d'une portion postérieure de ce dernier de diamètre interne supérieur à son diamètre interne courant.

Par ailleurs, de façon avantageuse, les portions déformables portant les premiers organes de blocage en translation consistent en deux pattes déformables radialement, s'étendant dans le prolongement postérieur de la bague, et sur chacune desquelles est disposé un cran de blocage postérieur.

De plus, chaque cran de blocage postérieur est avantageusement disposé en position intermédiaire sur la longueur de la patte, de sorte que se trouve ménagé à l'arrière desdits crans une rampe qui permet, en fin d'injection, tant qu'un effort est exercé sur le poussoir, d'absorber les variations de longueurs des seringues, et donc de garantir l'injection totale de la dose de liquide.

Par ailleurs, de façon avantageuse, le poussoir de la tige de piston présente la forme d'une coupelle dotée d'un bord à profil oblique, et chaque patte présente une tranche arrière à profil oblique complémentaire.

D'autres caractéristiques, buts et avantages de l'invention ressortiront de la description détaillée qui suit en référence aux dessins annexés qui en représentent à titre d'exemple non limitatif un mode de réalisation préférentiel. Sur ces dessins :
- la figure 1 est une coupe longitudinale par un plan axial d'un dispositif d'injection conforme à l'invention, représenté avant utilisation,
- la figure 2 est une vue en perspective de l'étui protecteur de ce dispositif d'injection,
- la figure 3 est une coupe longitudinale par un plan axial A de cet étui protecteur,
- la figure 4 est une coupe longitudinale par un plan axial B de cet étui protecteur,
- la figure 5 est une coupe transversale par un plan C de cet étui protecteur,
- la figure 6 est une coupe transversale par un plan D de cet étui protecteur,
- la figure 7 est une coupe longitudinale par un plan axial E de la bague de verrouillage de ce dispositif d'injection,
- la figure 8 est une vue en perspective de cette bague de verrouillage,
- la figure 9 est une coupe longitudinale par un plan axial F de l'embout à griffes de ce dispositif d'injection,
- la figure 10 est une vue en perspective de cet embout à griffes,
- les figures 11a à 11f sont des vues schématiques représentant les étapes successives de fabrication de ce dispositif d'injection,
- la figure 12 est une coupe longitudinale par un plan axial de ce dispositif d'injection représentant la phase initiale de retrait du capuchon de protection, en vue de l'utilisation dudit dispositif d'injection,
- la figure 13 est une coupe longitudinale par un plan axial représentant le dispositif d'injection en fin d'injection,
- et la figure 14 est une coupe longitudinale par un plan axial représentant le dispositif d'injection dans sa position de protection après usage.

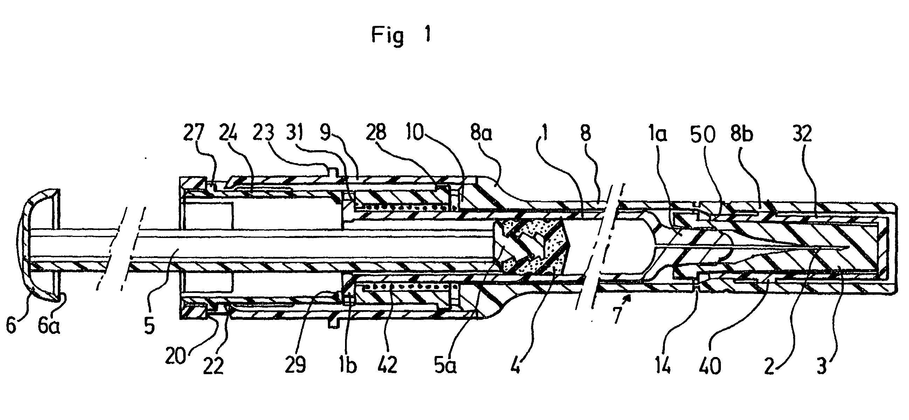

Le dispositif d'injection selon l'invention représenté à la figure 1 comprend, en premier lieu, une seringue pré-remplie 1 de type traditionnel, telle que par exemple réalisée en verre, comportant de façon classique un nez antérieur la sur lequel est montée une aiguille 2, et une collerette 1b au niveau de son extrémité postérieure.

Cette seringue 1 comporte également de façon classique un capuchon 3 de protection de l'aiguille 2 adapté pour être monté de façon étanche sur le nez antérieur 1a de ladite seringue.

Elle comprend également de façon classique un piston 4 délimitant la chambre remplie d'une dose de liquide, percé d'un alésage borgne taraudé dans lequel vient se visser l'extrémité filetée Sa d'une tige de piston 5 dotée au niveau de son extrémité opposée d'un poussoir 6.

Selon l'invention, ce poussoir 6 se présente sous la forme d'une coupelle présentant une tranche 6a à profil oblique à des fins explicitées plus loin.

Le dispositif d'injection selon l'invention comprend également un ensemble de protection de la seringue 1 adapté pour être entièrement pré-assemblé avant que ladite seringue initialement pré-remplie soit mise en place dans ledit ensemble de protection.

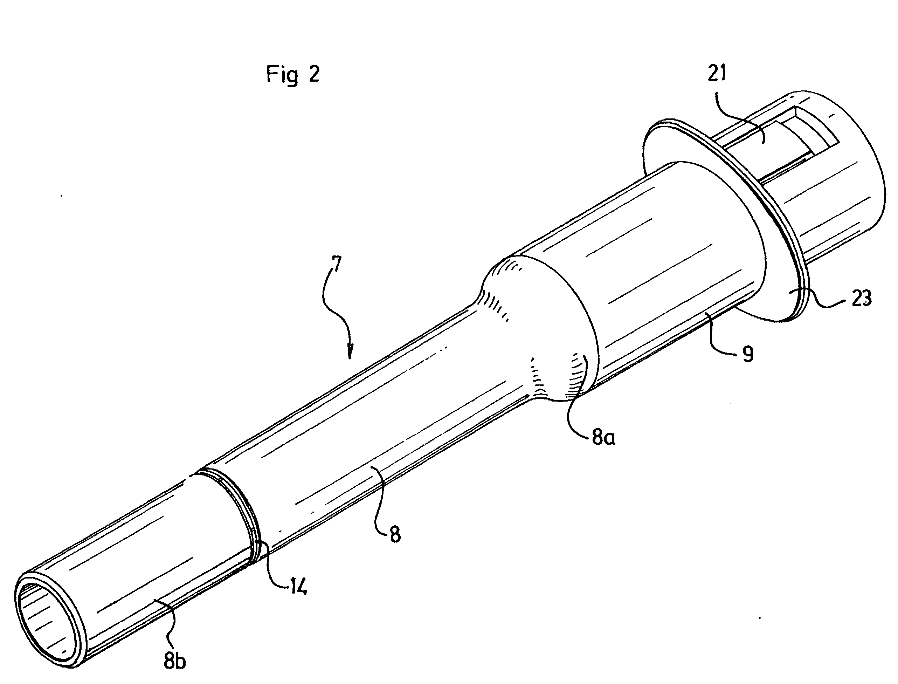

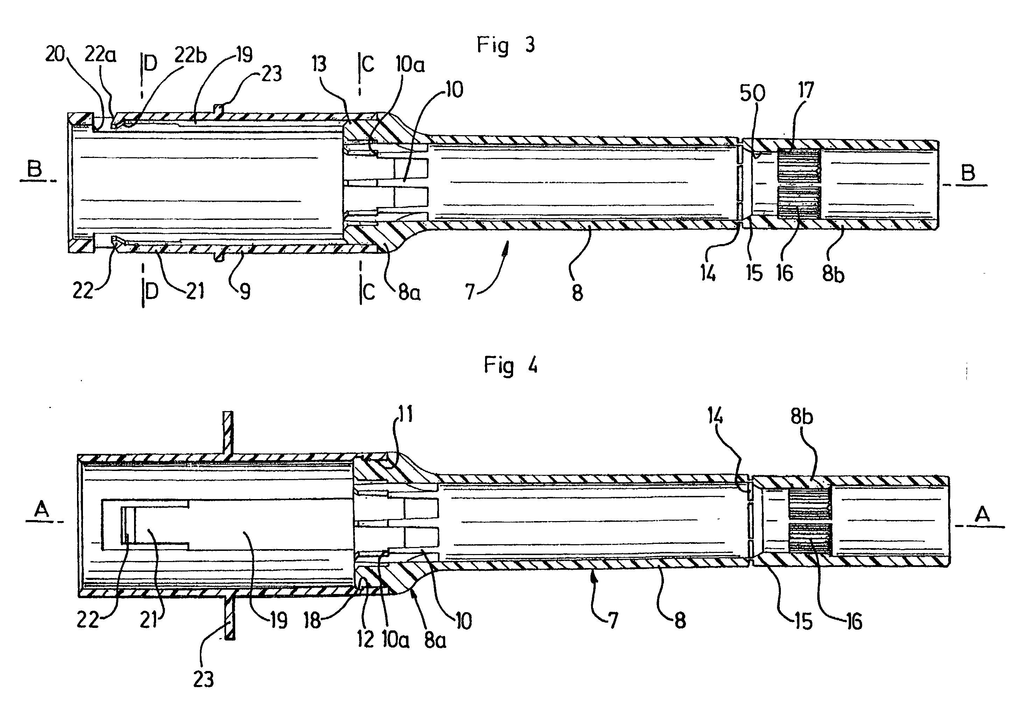

Cet ensemble de protection comprend en premier lieu un étui protecteur 7 représenté aux figures 2 à 6 composé de deux corps tubulaires antérieur 8 et postérieur 9 adaptés pour venir s'emmancher dans le prolongement l'un de l'autre.

Le corps antérieur 8 présente un diamètre interne conjugué du diamètre externe de la seringue 1 et une longueur adaptée pour loger l'aiguille 2 dotée de son capuchon de protection 3 et sensiblement 80% de la longueur de la seringue 1.

Au niveau de son extrémité postérieure, ce corps antérieur 8 comporte un tronçon postérieur 8a de forme externe sensiblement ovoïde de diamètres externes supérieurs au diamètre externe courant dudit corps antérieur, évidé intérieurement de façon à comporter des nervures longitudinales internes telles que 10 définissant un diamètre interne identique à celui du diamètre interne courant de ce corps antérieur 8.

Chacune de ces nervures 10 présente, en outre, un épaulement 10a délimitant une portion d'extrémité postérieure de diamètre interne sensiblement supérieur au diamètre de la seringue 1.

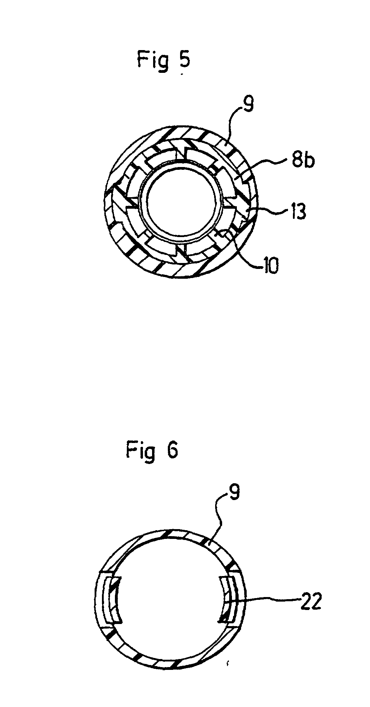

Côté externe, ce tronçon postérieur 8a comporte une gorge annulaire 11 délimitée par un jonc postérieur 12 de clipsage. De plus, tel que représenté à la figure 5, cette gorge 11 est interrompue par deux ergots tels que 13 diamétralement opposés faisant saillie radialement par rapport au jonc de clipsage 12.

Le corps anterieur 8 peut éventuellement également, au niveau de son extrémité antérieure, comporter un tronçon antérieur 8b sécable délimité par une zone annulaire frangible 14 positionnée de façon à se trouver sensiblement au niveau du nez antérieur la de la seringue 1 une fois celle-ci mise en place.

Ce tronçon sécable 8b présente intérieurement au niveau de son extrémité postérieure un profil oblique 15 formant une rampe délimitant une portion postérieure constituant un col 50 de diamètre sensiblement inférieur à celui du diamètre interne courant du corps antérieur 8.

A l'avant de cette portion postérieure, le tronçon sécable 8b comporte, en outre, une pluralité de crans longitudinaux tels que 16 répartis sur la périphérie de la face interne dudit tronçon sécable et délimitant un diamètre interne identique à celui de ladite portion postérieure, de sorte que les fonds desdits crans définissent un épaulement 17 avec l'extrémité antérieure de cette portion postérieure.

En dernier lieu, concernant ce tronçon sécable 8b, la portion antérieure de ce dernier située à l'avant des crans 16 présente un diamètre interne identique au diamètre séparant le fond des crans.

Le corps postérieur 9 présente quant à lui une forme sensiblement ovoïde conjuguée de celle du tronçon postérieur 8a du corps antérieur 8, adaptée pour venir s'emmancher sur ledit tronçon postérieur 8a.

A cet effet, ce corps postérieur 9 comporte, en premier lieu, une gorge annulaire interne 18 agencée pour loger le jonc de clipsage 12. Il comporte, en outre, deux rainures longitudinales internes telle que 19 diamétralement opposées, aptes à loger chacune un ergot 13, de façon à assurer un blocage en rotation des deux corps 8, 9, lesdites rainures étant interrompues à faible distance de l'extrémité postérieure de ce corps postérieur 9, de façon que ce dernier présente un épaulement interne 20 au droit de l'extrémité de ces rainures 19.

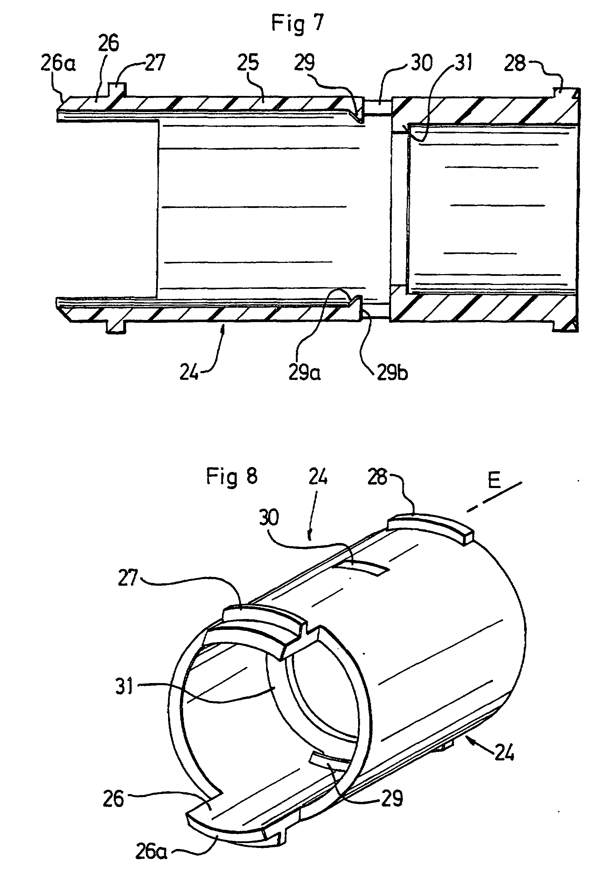

Tel que représenté à la figure 7, les rainures 19 sont ménagées selon le diamètre le plus grand du corps postérieur 9, de façon à minimiser l'épaisseur de la paroi dudit corps postérieur.

Le corps postérieur 9 présente, en outre, deux languettes déformables telles que 21 ménagées chacune dans une rainure 19 au niveau de l'extrémité postérieure de cette dernière et formées chacune par une découpe en forme de U ménagée dans la paroi dudit corps postérieur.

Chacune de ces languettes 21 comporte au niveau de son extrémité postérieure un crochet transversal 22 faisant saillie à l'intérieur du corps postérieur 9. Chacun de ces crochets comporte une face postérieure anti-retour 22a sensiblement radiale, et une face antérieure oblique 22b formant une rampe.

En dernier lieu, le corps postérieur 9 comporte une collerette externe classique 23 d'appui digital.

L'ensemble de protection comprend, en second lieu, une bague de verrouillage 24 de forme adaptée pour venir s'insérer dans le corps postérieur 9, en étant présentée en regard de la face antérieure de ce dernier.

Cette bague de verrouillage 24 de longueur adaptée pour s'insérer dans le corps postérieur 9 se présente sous la forme d'un manchon cylindrique 25 prolongé postérieurement par deux pattes telles que 26 diamétralement opposées en forme de secteur cylindrique.

Chacune de ces deux pattes 26 présente, en premier lieu, une face d'extrémité postérieure 26a à profil oblique complémentaire de celui de la tranche 6a du poussoir 6 de la tige de piston 5.

Chacune de ces pattes 26 comporte, en outre, sensiblement à mi-longueur, une nervure externe transversale 27, adaptée pour pouvoir coulisser dans une des rainures 10 du corps postérieur 9.

Le manchon cylindrique 25 de cette bague de verrouillage 24 comporte, quant à lui, axées, sur les mêmes génératrices que les nervures transversales 27, deux nervures transversales telles que 28 également adaptées pour coulisser dans les rainures 10 du corps postérieur 9, et ménagées au niveau de l'extrémité antérieure dudit manchon.

Ce manchon cylindrique 25 comprend également sensiblement à mi-longueur et dans l'alignement axial des nervures 27, 28 précitées, deux nervures internes telles que 29 diamétralement opposées en aval de chacune desquelles la paroi périphérique dudit manchon est percée d'une lumière telle que 30 permettant le démoulage de la contre-dépouille.

Chacune de ces nervures internes 29 présente une face postérieure 29a à profil oblique formant rampe et une face antérieure 29b radiale de blocage anti-retour.

Enfin, le manchon cylindrique 25 comprend en aval des lumières 30 et à une distance des nervures internes 29 conjuguée de l'épaisseur de la collerette 1b de la seringue 1, un épaulement interne annulaire 31.

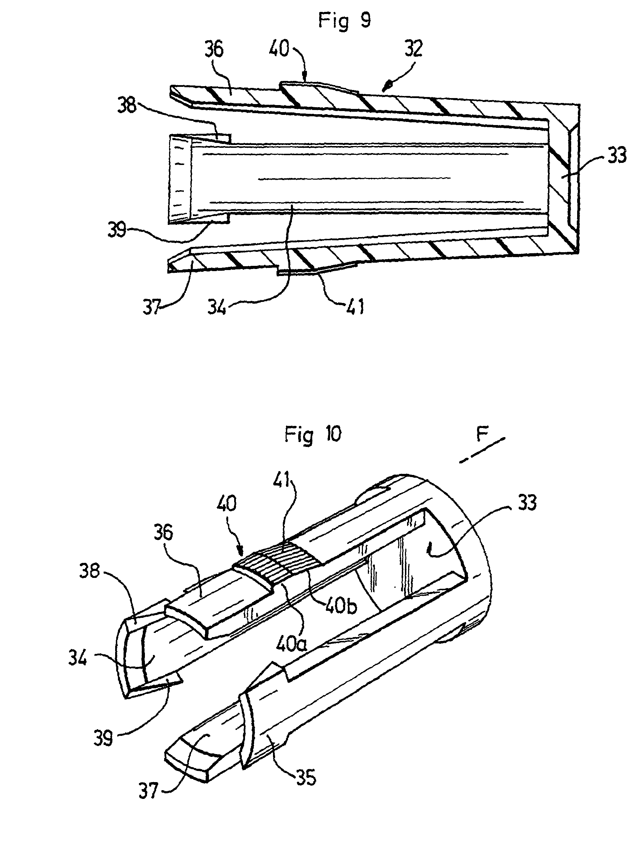

Le dispositif d'injection peut éventuellement également inclure un embout à griffes 32 représenté aux figures 9 et 10 destiné à venir coiffer le capuchon de protection 3 et à entraîner le retrait de ce dernier après rupture de la zone frangible 14 du corps antérieur 8 de l'étui protecteur 7.

Cet embout à griffes 32 présente une paroi frontale cylindrique 33, de diamètre adapté pour pénétrer dans le tronçon sécable 8b, en périphérie de laquelle s'étendent sensiblement orthogonalement quatre pattes longitudinales distinctes 34, 35, 36, 37 uniformément réparties par rapport à l'axe de ladite paroi :
- deux pattes 34, 35 diamétralement opposées dotées au niveau de leur extrémité libre chacune de deux griffes latérales telles que 38, 39 adaptées pour pouvoir pénétrer dans le capuchon de protection 3,
- deux autres pattes 36, 37 diamétralement opposées présentant, en position intermédiaire de leur longueur, chacune un bossage externe tel que 40 présentant une face externe pourvue de crans longitudinaux tels que 41 conjugués des crans 16 du tronçon sécable 8b. De plus, chaque bossage 40 présente une portion postérieure cylindrique 40a précédée d'une portion antérieure inclinée 40b formant rampe.

Il est à noter, en outre, que tel que représenté à la figure 9, lors du moulage, les pattes 34-37 sont réalisées en position sensiblement "ouverte", c'est-à-dire inclinées vers l'extérieur par rapport à l'axe de la paroi frontale 33.

La fabrication du dispositif d'injection ci-dessus décrit ainsi que l'interconnexion des divers organes des éléments constitutifs est explicitée ci-dessous en référence aux figures 11a à 11f.

La première étape consiste à introduire la bague de verrouillage 24 à l'intérieur du corps postérieur 9 de l'étui protecteur 7, en présentant cette dernière en regard de la face antérieure dudit corps postérieur, jusqu'à amener les nervures 27 en butée contre l'épaulement 20 (figure 11a). Il est à noter que cette mise en place est autorisée par l'élasticité des languettes 21 et à la forme de la face antérieure 22b des nervures 22 qui forme une rampe autorisant le franchissement desdites nervures.

Il est également à noter qu'une fois la bague de verrouillage 24 mise en place, celle-ci est bloquée en rotation relativement au corps postérieur 9 du fait du positionnement des nervures 27 dans les rainures 19.

La deuxième étape consiste à introduire partiellement un ressort à spirale 42 à l'intérieur de la bague de verrouillage 24 en présentant ce dernier en regard de la face antérieure de ladite bague, jusqu'à amener une de ses extrémités en butée contre l'épaulement 31 (figure 11b).

La troisième étape consiste à venir emmancher le corps antérieur 8 sur le corps postérieur par coopération du jonc de clipsage 12 avec la gorge 18 (figure 11c). Au cours de cette opération, le ressort 42 se trouve automatiquement comprimé entre les épaulements 10a des nervures 10 et l'épaulement 31. De plus, les corps antérieur 8 et postérieur se trouvent bloqués en rotation relative du fait du positionnement des ergots 13 dans les rainures 19.

Au terme de ces trois opérations qui peuvent être facilement automatisées, on obtient un ensemble de protection entièrement pré-assemblé, à l'intérieur duquel peut ensuite être introduite la seringue pré-remplie 1 tel qu'explicité ci-dessous.

Préalablement à cette introduction, et tel que représenté à la figure 11d, l'embout à griffes 32 est positionné sur le capuchon de protection 3 de la seringue 1, à ce stade dépourvue de tige de piston 5. Lors de ce positionnement, les pattes 34-37 de l'embout à griffes 32 viennent uniquement se positionner autour du capuchon de protection 3, sans risque d'enfoncer ce dernier et d'endommager l'aiguille 2 et/ou de détruire l'étanchéité.

La seringue 1 munie de l'embout à griffes 32 est ensuite introduite dans le corps postérieur 9 de l'étui protecteur 7 jusqu'à amener la collerette 1b à se bloquer entre les nervures 29 et l'épaulement 31 (figure 11e). Il est à noter que cette introduction est autorisée par la faculté de se déformer des nervures 29 et à la forme de rampe de la face postérieure 29a de ces nervures 29 qui autorise le franchissement de ces dernières par la collerette 1b.

De plus, lors de cette introduction, l'embout à griffes 32 est amené à se resserrer sur le capuchon de protection 3 lors du passage des bossages 40 de ce dernier au niveau de la rampe 15 du tronçon sécable 8b et du col 50, passage en outre facilité par la forme de rampe de la portion antérieure 40b desdits bossages.

Il est à noter, en outre, que tel que représenté à la figure 1, lors de ce resserrement, les griffes 38, 39 viennent pénétrer dans le capuchon de protection 3 en aval du cône en verre dont sont munis classiquement les nez antérieurs des seringues classiques 1, destinées à assurer l'étanchéité. Ainsi, tout risque d'enfoncement du capuchon de protection 3 et donc d'endommagement de l'aiguille 2 et/ou de destruction de l'étanchéité se trouve écarté.

Une fois cette étape réalisée, il convient de noter que l'embout à griffes 32 et l'étui protecteur 7 sont bloqués en rotation relative du fait de la coopération des crans respectifs 16, 41 de ces derniers. De plus, d'une part, le capuchon de protection 3 est bloqué en rotation par rapport à l'embout à griffes 32 du fait de la pénétration dans ce dernier des griffes 38, 39, et d'autre part, cet embout à griffes 32, l'étui protecteur 7 et la bague de verrouillage 24 sont également bloqués en rotation relative tel qu'explicité ci-dessus.

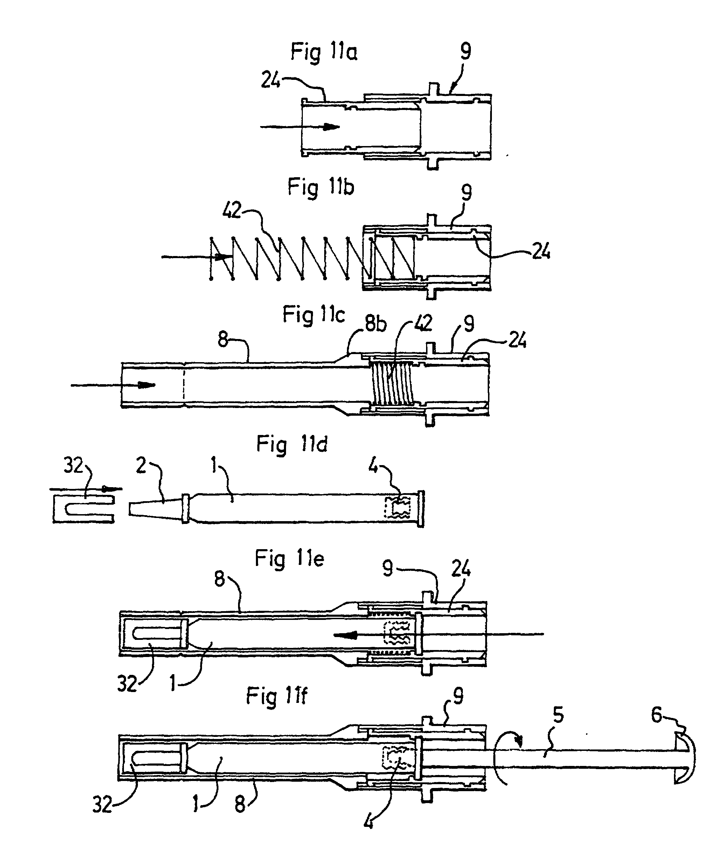

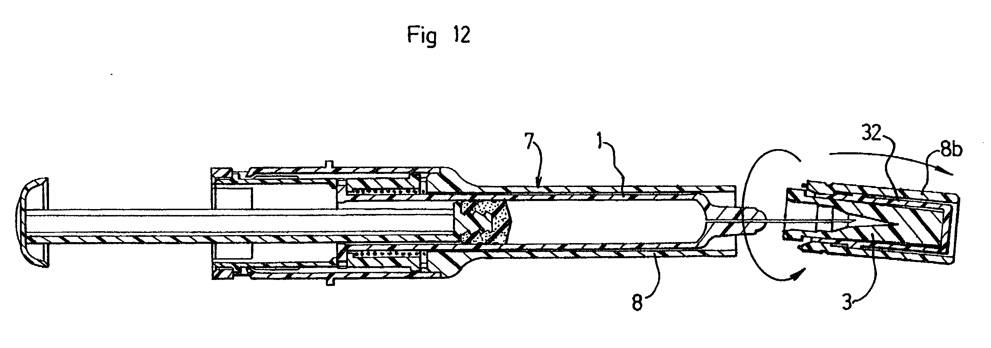

La dernière étape représentée à la figure 11f consiste enfin à solidariser de façon classique la tige de piston 5 au piston 4. On obtient alors un dispositif d'injection prêt à l'emploi dont l'utilisation est décrite ci-après en référence aux figures 12 à 14.

En premier lieu, tel que représenté à la figure 12, l'étape initiale consiste à rompre la zone frangible 14 en soumettant de façon classique le tronçon sécable 8 à un mouvement de rotation et en tirant celui-ci. Lors du mouvement, l'ensemble des éléments (capuchon de protection3, étui protecteur 7, bague de verrouillage 24) étant bloqués relativement en rotation, le capuchon de protection 3 est amené en premier lieu à tourner, facilitant le décollage de ce dernier, puis l'embout à griffes 32 est bloqué en translation par rapport au tronçon sécable 8b par butée des bossages 40 contre l'épaulement 17, de sorte que le capuchon de protection 3 est retiré simultanément avec l'embout à griffes 32 et ledit tronçon sécable.

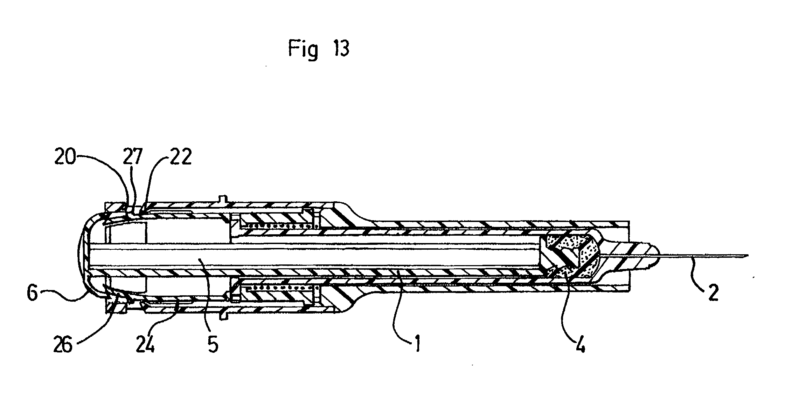

L'injection peut alors être réalisée de façon classique par action antagoniste sur le poussoir 6 et la collerette d'appui digital 23. En fin d'injection, tel que représenté à la figure 13, le bord profilé 6a du poussoir 6 vient coopérer avec le bord profilé 26a des pattes 26 de la bague de verrouillage 24, conduisant à déformer radialement vers l'intérieur lesdites pattes 26 jusqu'à entraîner la libération des nervures 27.

Il est à noter, en outre, que les nervures 27 étant disposées en position intermédiaire sur les pattes 26, ces dernières possèdent ainsi une rampe postérieure auxdites nervures qui permet d'absorber les tolérances de fabrication des seringues 1 et de garantir la délivrance de l'intégralité de la dose de liquide.

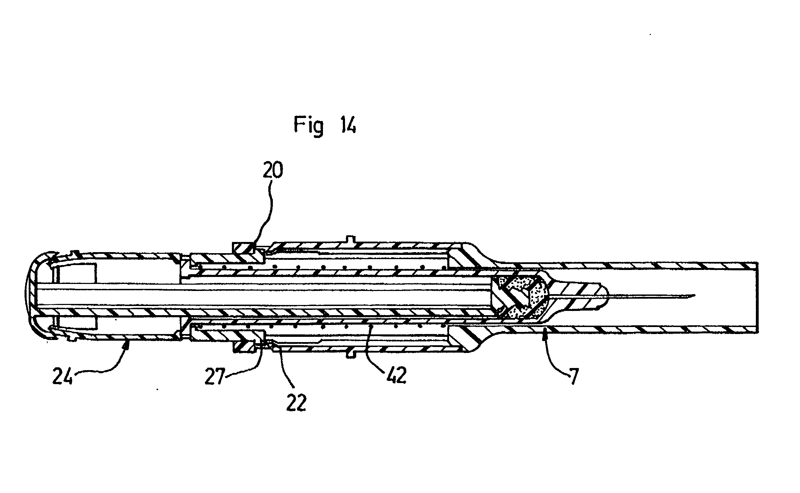

Tel que représenté à la figure 14, une fois l'injection terminée et l'effort sur le poussoir 6 supprimé, la bague de verrouillage 24 sollicitée par le ressort 42 est repoussée à l'intérieur de l'étui protecteur 7 entraînant la seringue 1, jusqu'à ce que les nervures 28 franchissent la rampe 22b des nervures 22 et viennent se bloquer entre lesdites nervures 22 et l'épaulement 20, interdisant un emploi ultérieur du dispositif d'injection.

En dernier lieu, et de façon avantageuse, le corps antérieur 8 de l'étui protecteur 7 est réalisé en un matériau translucide de façon à permettre de visualiser la seringue 1. Le corps postérieur 9 de cet étui protecteur est quant à lui réalisé en un matériau opaque de façon à masquer à la vue de l'utilisateur et du patient le mécanisme de déclenchement.

La bague de verrouillage 24 est quant à elle réalisée en un matériau de couleur différente de celui du corps postérieur 9 de façon à permettre d'identifier immédiatement les dispositifs d'injection déjà utilisés.

## Revendications

1. Dispositif d'injection à usage unique comprenant une seringue (1) dotée d'un nez antérieur (1a) portant une aiguille d'injection (2) et d'une collerette postérieure (1b), ladite seringue délimitant une chambre remplie d'une dose de liquide à injecter, obturée par un piston (4) solidarisé à une des extrémités d'une tige de piston (5) dotée d'un poussoir (6) au niveau de son extrémité opposée, ledit dispositif d'injection étant **caractérisé en ce qu'**il comprend un dispositif de protection après usage de la seringue (1) comportant :
- un étui protecteur (7) composé de deux corps tubulaires (8, 9), dits corps antérieur et corps postérieur, dotés de moyens d'assemblage (12, 18) aptes à permettre de les solidariser dans le prolongement l'un de l'autre, le corps antérieur (8) présentant un diamètre conjugué de celui de la seringue (1), et une longueur adaptée pour loger partiellement ladite seringue, et le corps postérieur (9) présentant un diamètre supérieur à celui de la collerette (1b) de ladite seringue, et comportant des organes d'appui-doigts (23),
- une bague (24) disposée dans le corps postérieur (9) et comportant des moyens (29, 31) de blocage de la collerette (1b) de la seringue (1) aptes à permettre d'introduire ladite seringue (2) en présentant l'aiguille en regard du corps postérieur (9) de l'étui protecteur (7),
- la bague (24) et le corps postérieur (9) comportant :
. des premiers moyens de blocage relatif en translation (20, 22, 27) aptes à permettre d'introduire ladite bague dans ledit corps postérieur en la présentant en regard de la face d'assemblage de ce dernier avec le corps antérieur (8), et définissant une première position de blocage, dite d'injection, permettant l'injection une fois le dispositif d'injection assemblé,
. des deuxièmes moyens de blocage relatif en translation (20, 22, 28) définissant une deuxième position de blocage, dite de protection après usage, obtenue après coulissement vers l'arrière de la bague (24) à l'intérieur du corps postérieur (9), dans laquelle l'aiguille d'injection (2) se trouve logée dans le corps antérieur (8),
- la bague (24) et le corps antérieur (8) étant dotés de moyens de butée (10a, 31) aptes à maintenir des moyens élastiques (42) comprimés dans la première position de blocage d'injection,
- et la bague (24) ou le corps postérieur (9) comportant une portion postérieure (26) déformable radialement et portant les premiers moyens de blocage en translation (27), agencée pour être déformée par le poussoir (6) de la tige de piston (5) en fin de course de cette dernière, de façon à libérer lesdits premiers organes de blocage et à autoriser le déplacement de la bague (24) vers sa deuxième position de blocage de protection après usage.

2. Dispositif d'injection selon la revendication 1, **caractérisé en ce que** la bague (24) est réalisée en un matériau de couleur différente de celui de l'étui protecteur (7).

3. Dispositif d'injection selon l'une des revendications 1 ou 2, **caractérisé en ce que** le corps antérieur (8) de l'étui protecteur (7) est réalisé en un matériau translucide.

4. Dispositif d'injection selon l'une des revendications 1 à 3, **caractérisé en ce que** le corps postérieur (9) de l'étui protecteur (7) est réalisé en un matériau opaque.

5. Dispositif d'injection selon l'une des revendications précédentes, **caractérisé en ce que** les moyens d'assemblage des corps antérieur (8) et postérieur (9) de l'étui protecteur (7) comprennent une gorge annulaire (18) interne ménagée dans le corps postérieur (9) et un jonc de clipsage externe (12) ménagé sur le corps antérieur (8).

6. Dispositif d'injection selon l'une des revendications précédentes, **caractérisé en ce que** les moyens de blocage en translation de la bague (24) et du corps postérieur (9) de l'étui protecteur (7) comprennent :
- au moins une rainure longitudinale interne (19) ménagée dans le corps postérieur (9), prolongée par une cavité (20, 22) de blocage postérieure,
- pour chaque rainure (19), deux crans de blocage postérieur (27) et antérieur (28) ménagés sur la bague (24) et adaptés pour coulisser dans ladite rainure et se loger dans la cavité de blocage.

7. Dispositif d'injection selon la revendication 6, **caractérisé en ce que** chaque cavité de blocage consiste en une lumière ménagée dans la paroi périphérique du corps postérieur (9), délimitée postérieurement par un épaulement interne (20) et antérieurement par un cran interne (22) en forme de dent asymétrique dotée d'une face antérieure (22b) inclinée, et portée par une languette déformable (21) ménagée dans ladite paroi périphérique du corps postérieur (9).

8. Dispositif d'injection selon l'une des revendications 6 ou 7, **caractérisé en ce que** le corps antérieur (8) de l'étui protecteur (7) comporte, pour chaque rainure (19) du corps postérieur (9), un ergot externe (13) apte à se loger dans ladite rainure.

9. Dispositif d'injection selon l'une des revendications précédentes, **caractérisé en ce que** les moyens de blocage de la collerette (1b) de la seringue (1) comprennent au moins un cran interne postérieur (29) ménagé dans la bague (24) et consistant en une dent asymétrique dotée d'une face postérieure inclinée (29a), et un épaulement interne antérieur (31) ménagé dans la bague (24) à une distance de chaque cran (29) équivalent à l'épaisseur de ladite collerette.

10. Dispositif d'injection selon la revendication 9, **caractérisé en ce que** les moyens de butée des moyens élastiques (42) comprennent l'épaulement (31) ménagé dans la bague (24) et un épaulement interne (10a) ménagé dans le corps antérieur (8) au niveau d'une portion postérieure (8a) de ce dernier de diamètre interne supérieur à son diamètre interne courant.

11. Dispositif d'injection selon l'une des revendications 6 ou 7, **caractérisé en ce que** les portions déformables portant les premiers organes de blocage en translation (27) consistent en deux pattes (26) déformables radialement, s'étendant dans le prolongement postérieur de la bague (24), et sur chacune desquelles est disposé un cran de blocage postérieur (27).

12. Dispositif d'injection selon la revendication 11, **caractérisé en ce que** chaque cran de blocage postérieur (27) est disposé en position intermédiaire sur la longueur de la patte (26).

13. Dispositif d'injection selon l'une des revendications 11 ou 12, **caractérisé en ce que** le poussoir (6) de la tige de piston (5) présente la forme d'une coupelle dotée d'un bord (6a) à profil oblique, chaque patte (26) présentant une tranche arrière (26a) à profil oblique complémentaire.

## Patentansprüche

1. Injektionsvorrichtung für die einmalige Verwendung, umfassend eine Spritze (1), die mit einer eine Injektionsnadel (2) tragenden vorderen Nase (1a) und einem hinteren Kragen (1b) versehen ist, wobei die genannte Spritze eine Kammer begrenzt, die mit einer Dosis einer zu injizierenden Flüssigkeit gefüllt und mit einem Kolben (4) verschlossen ist, der an einem seiner Enden mit einer Kolbenstange (5) einstückig ausgebildet ist und an seinem gegenüberliegenden Ende einen Vordrücker (6) aufweist, wobei die genannte Injektionsvorrichtung **dadurch gekennzeichnet ist, dass** sie eine Vorrichtung zum Schützen nach dem Gebrauch der Spritze (1) umfasst, die folgendes umfasst:
- eine Schutzhülse (7) aus zwei - einem vorderen und einem hinteren - röhrenförmigen Körpern (8, 9), die mit Verbindungsmitteln (12, 18) ausgestattet sind, die so gestaltet sind, dass sie in ihrer Verlängerung miteinander verbunden werden können, wobei der vordere Körper (8) einen Durchmesser aufweist, der mit dem der Spritze (1) gekoppelt ist, und eine Länge, die teilweise von der genannten Spritze aufgenommen wird, und wobei der hintere Körper (9) einen Durchmesser hat, der größer ist als der des Kragens (1b) der genannten Spritze, und Fingeranlageteile (23) aufweist,
- einen Ring (24), der im hinteren Körper (9) angeordnet ist und Mittel (29, 31) zum Sperren des Kragens (1b) der Spritze (1) aufweist, um.das Einführen der genannten Spritze (2) zuzulassen, so dass sich die Nadel beim hinteren Körper (9) der Schutzhülse (7) befindet wird,
- wobei der Ring (24) und der hintere Körper (9) folgendes umfassen:
. erste relative Translationssperrmittel (20, 22, 27), die so gestaltet sind, dass sie das Einführen des genannten Rings in den genannten hinteren Körper zulassen, so dass sich der Ring bei der Verbindungsfläche des Letzteren mit dem vorderen Körper (8) befindet, und unter Definition einer ersten Sperrposition, Injektionsposition genannt, die eine Injektion zulässt, wenn die Injektionsvorrichtung zusammengesetzt ist,
. zweite relative Translationssperrmittel (20, 22, 28), die eine zweite Sperrposition definieren, Nach-Gebrauch-Schutzposition genannt, die nach dem Schieben zum hinteren Teil des Rings (24) im Inneren des hinteren Körpers (9) eingenommen wird und in der die genannte Injektionsnadel (2) im vorderen Körper (8) aufgenommen wird,
- wobei der Ring (24) und der vordere Körper (8) mit Anschlagmitteln (10a, 31) versehen sind, um elastische Mittel (42) in der ersten Injektionssperrposition zusammengedrückt zu halten,
- und wobei der Ring (24) oder der hintere Körper (9) einen hinteren Abschnitt (26) aufweisen, der radial verformbar ist und die ersten Translationssperrmittel (27) trägt und so gestaltet ist, dass er vom Vordrücker (6) der Kolbenstange (5) am Ende des Weges des Letzteren verformt werden kann, um die genannten ersten Sperrorgane freizugeben und eine Verschiebung des Rings (24) in Richtung auf seine zweite Nachgebrauchsschutz-Sperrposition zuzulassen.

2. Injektionsvorrichtung nach Anspruch 1, **dadurch gekennzeichnet, dass** der Ring (24) aus einem Material mit einer Farbe hergestellt ist, die sich von der der Schutzhülse (7) unterscheidet.

3. Injektionsvorrichtung nach einem der Ansprüche 1 oder 2, **dadurch gekennzeichnet, dass** der vordere Körper (8) der Schutzhülse (7) aus einem lichtdurchlässigen Material hergestellt ist.

4. Injektionsvorrichtung nach einem der Ansprüche 1 bis 3, **dadurch gekennzeichnet, dass** der hintere Körper (9) der Schutzhülse (7) aus einem opaken Material hergestellt ist.

5. Injektionsvorrichtung nach einem der vorherigen Ansprüche, **dadurch gekennzeichnet, dass** die Verbindungsmittel des vorderen (8) und des hinteren (9) Körpers der Schutzhülse (7) eine interne ringförmige Auskehlung (18) aufweisen, die im hinteren Körper (9) angeordnet ist, sowie einen äußeren Klemmring (12), der auf dem vorderen Körper (8) angeordnet ist.

6. Injektionsvorrichtung nach einem der vorherigen Ansprüche, **dadurch gekennzeichnet, dass** die Translationssperrmittel des Rings (24) und des hinteren Körpers (9) der Schutzhülse (7) folgendes umfassen:
- wenigstens eine interne Längsrille (19), die im hinteren Körper (9) ausgebildet ist und durch einen hinteren Sperrhohlraum (20, 22) verlängert wird,
- für jede Rille (19) zwei hintere (27) und vordere (28) Rastzungen, die auf dem Ring (24) angeordnet und so gestaltet sind, dass sie in der genannten Rille gleiten und. in dem Sperrhohlraum aufgenommen werden.

7. Injektionsvorrichtung nach Anspruch 6, **dadurch gekennzeichnet, dass** jeder Sperrhohlraum aus einem Schlitz besteht, der in der Umfangswand des hinteren Körpers (9) ausgebildet ist, hinten durch einen Innenansatz (20) und vorne durch eine Innenzunge (22) in der Form eines asymmetrischen Zahns begrenzt wird, der von einer vorderen geneigten Fläche (22b) gebildet wird, und der von einer verformbaren Lasche (21) getragen wird, die in der genannten Umfangswand des hinteren Körpers (9) ausgebildet ist.

8. Injektionsvorrichtung nach einem der Ansprüche 6 oder 7, **dadurch gekennzeichnet, dass** der vordere Körper (8) der Schutzhülse (7) für jede Rille (19) des hinteren Körpers (9) einen äußeren Vorsprung (13) aufweist, der so gestaltet ist, dass er von der genannten Rille aufgenommen wird.

9. Injektionsvorrichtung nach einem der vorherigen Ansprüche, **dadurch gekennzeichnet, dass** die Sperrmittel des Kragens (1b) der Spritze (1) wenigstens eine hintere Innenzunge (29), die im Ring (24) ausgebildet ist und aus einem mit einer geneigten hinteren Fläche (29a) ausgestatteten asymmetrischen Zahn besteht, und einen vorderen Innenansatz (31) aufweisen, der im Ring (24) in einem Abstand von jeder Zunge (29) vorgesehen ist, die der Dicke des genannten Kragens entspricht.

10. Injektionsvorrichtung nach Anspruch 9, **dadurch gekennzeichnet, dass** die Anschlagmittel der elastischen Mittel (42) den Ansatz (31) umfassen, der im Ring (24) vorhanden ist, und einen Innenansatz (10a), der im vorderen Körper (8) in der Höhe eines hinteren Abschnittes (8a) des Letzteren mit einem Innendurchmesser vorgesehen ist, der größer ist als sein laufender Innendurchmesser.

11. Injektionsvorrichtung nach einem der Ansprüche 6 oder 7, **dadurch gekennzeichnet, dass** die verformbaren Abschnitte erste Translationssperrorgane (27) aufweisen, die aus zwei radial verformbaren Klauen (26) bestehen, die in der hinteren Verlängerung des Rings (24) verlaufen und auf denen jeweils eine hintere Rastzunge (27) angeordnet ist.

12. Injektionsvorrichtung nach Anspruch 11, **dadurch gekennzeichnet, dass** jede hintere Rastzunge (27) in einer Zwischenposition auf der Länge der Klaue (26) angeordnet ist.

13. Injektionsvorrichtung nach einem der Ansprüche 11 oder 12, **dadurch gekennzeichnet, dass** der Vordrücker (6) der Kolbenstange (5) die Form einer Kuppel hat, die einen Rand (6a) mit schrägem Profil aufweist, wobei jede Klaue (26) eine hintere Scheibe (26a) mit einem ergänzenden schrägen Profil aufweist.

## Claims

1. Single-use device for injection comprising a syringe (1) which is provided with a front nose (1a) which supports an injection needle (2), and with a rear collar (1b), the said syringe delimiting a chamber which is filled with a dose of fluid to be injected, and is sealed by a piston (4) which is rendered integral with one of the ends of a piston rod (5), which is provided with a thruster (6) at its opposite end, the said device for injection being **characterised in that** it comprises a device for protection after the syringe (1) has been used, comprising:
- a protective sheath (7), consisting of two tubular bodies (8, 9), which are known as the front body and the rear body, and are provided with assembly means (12, 18) which make it possible to render the bodies integral one in the extension of the other, the front body (8) having a diameter which is conjugated relative to that of the syringe (1), and a length which is designed to accommodate the said syringe partially, and the rear body (9) having a diameter which is larger than that of the collar (1b) of the said syringe, and comprising finger-support units (23);
- a ring (24) which is disposed in the rear body (9), which comprises means (29, 31) for locking the collar (1b) of the syringe (1), and can make it possible to introduce the said syringe (2), by presenting the needle opposite the rear body (9) of the protective sheath (7);
- the ring (24) and the rear body (9) comprising:
. first means (20, 22, 27) for relative locking in translation, which can make it possible to introduce the said ring in the said rear body, by presenting the ring opposite the surface of assembly of the latter with the front body (8), and defining a first locking position, known as the position for injection, which allows the injection to take place when the device for injection has been assembled;
. second means (20, 22, 28) for relative locking in translation, which define a second locking position, known as the position for protection after use, which is obtained after rearward sliding of the ring (24) inside the rear body (9), in which the injection needle (2) is accommodated in the front body (8);
- the ring (24) and the front body (8) being provided with stop means (10a, 31), which can keep resilient means (42) compressed in the first locking position, for injection; and
- the ring (24) or the rear body (9) comprising a rear portion (26) which is deformable radially, and supporting the first locking means (27) in translation, which portion is designed to be. deformed by the thruster (6) of the piston rod (5) at the end of the path of the latter, such as to release the said first locking units, and to permit displacement of the ring (24) towards its second locking position, for protection after use.

2. Device for injection according to claim 1,
**characterised in that** the ring (24) is made of a material which has a colour different from that of the protective sheath (7).

3. Device for injection according to claim 1 or claim 2, **characterised in that** the front body (8) of the protective sheath (7) is made of a translucent material.

4. Device for injection according to any one of claims 1 to 3, **characterised in that** the rear body (9) of the protective sheath (7) is made of an opaque material.

5. Device for injection according to any one of the preceding claims, **characterised in that** the means for assembly of the front body (8) and rear body (9) of the protective sheath (7) comprise an inner annular groove (18) which is provided in the rear body (9), and an outer clip ring (12) which is provided on the front body (8).

6. Device for injection according to any one of the preceding claims, **characterised in that** the means for locking in translation of the ring (24) and of the rear body (9) of the protective sheath (7) comprise:
- at least one inner longitudinal groove (19) which is provided in the rear body (9), and is extended by a arear locking cavity (20, 22) ;
- for each groove (19), two, rear (27) and front (28)
- locking catches, which are provided on the ring (24), and are designed to slide in the said groove, and to be accommodated in the locking cavity.

7. Device for injection according to claim 6,
**characterised in that** each locking cavity consists of an aperture which is provided in the peripheral wall of the rear body (9), and is delimited at the rear by an inner shoulder (20), and at the front by an inner catch (22) in the form of an asymmetrical tooth, which is provided with an inclined front surface (22b), and.is supported by a deformable tab (21) which is provided in the said peripheral wall. of the rear body (9).

8. Device for injection according to claim 6 or claim 7, **characterised in that** for each groove (19) in the rear body (9), the front body (8) of the protective sheath (7) comprises an outer lug (13), which can be accommodated in the said groove.

9. Device for injection according to any one of the preceding claims, **characterised in that** the means for locking the collar (1b) of the syringe (1). comprise at least one rear inner catch (29), which is provided in the ring (24), and consists of an asymmetrical tooth, which is provided with an inclined rear surface (29a), and a front inner shoulder (31), which is provided in the ring (24), at a distance from each catch (29) which is equivalent to the thickness of the said collar.

10. Device for injection according to claim 9,
**characterised in that** the stop means of the resilient means (42) comprise the shoulder (31) which is provided in the ring (24), and an inner shoulder (10a) which is provided in the front body (8), at a rear portion (8a) of the latter, which has an inner diameter which is larger than its regular inner diameter.

11. Device for injection according to claim 6 or claim 7, **characterised in that** the deformable portions which support the first units (27) for locking in translation consist of two tabs (26) which can be deformed radially, and extend in the rear extension of the ring (24), and on each of which there is disposed a rear locking catch (27).

12. Device for injection according to claim 11, **characterised in that** each rear locking catch (27) is disposed in an intermediate position along the length of the tab (26).

13. Device for injection according to claim 11 or claim 12, **characterised in that** the thruster (6) of the piston rod (5) has the shape of a bowl which is provided with an edge (6a) with an oblique profile, and each tab (26) has a rear section (26a) with a complementary oblique profile.
